(19) Europäisches Patentamt
European Patent Office
Office européen des brevets

(11) Numéro de publication: **0 099 770**
**B1**

# (12) FASCICULE DE BREVET EUROPEEN

(45) Date de publication du fascicule du brevet: **27.08.86**

(51) Int. Cl.[4]: **C 07 D 513/04,** A 61 K 31/54 // (C07D513/04, 279:00, 265:00)

(21) Numéro de dépôt: **83401168.6**

(22) Date de dépôt: **08.06.83**

(54) **Nouveaux dérivés d'oxazinobenzothiazine-6,6-dioxyde, leur préparation et leur application en tant que médicaments.**

(30) Priorité: **15.06.82 FR 8210409**

(43) Date de publication de la demande: **01.02.84 Bulletin 84/5**

(45) Mention de la délivrance du brevet: **27.08.86 Bulletin 86/35**

(84) Etats contractants désignés: **AT BE CH DE FR GB IT LI LU NL SE**

(56) Documents cités:
**US - A - 3 923 801**

**JOURNAL OF MEDICINAL CHEMISTRY, vol. 16, no. 1, 1er janvier 1973, pages 44-48, Columbus, Ohio, USA, H. ZINNES et al.: "1,2-benzothiazines. 6. 3-carbamoyl-4-hydroxy-2H-1,2-benzothiazine 1,1-dioxides as antiinflammatory agents"**

**Le dossier contient des informations techniques présentées postérieurement au dépôt de la demande et ne figurant pas dans le présent fascicule.**

(73) Titulaire: **PROVESAN S.A., 1, place St Gervais, CH-1211 Genève (CH)**

(72) Inventeur: **Esteve Soler, José, Via Augusta 244, Barcelone (ES)**

(74) Mandataire: **Ahner, Francis et al, CABINET REGIMBEAU 26, avenue Kléber, F-75116 Paris (FR)**

**EP 0 099 770 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés d'oxazinobenzothiazine-6,6-dioxyde, leur procédé de préparation, ainsi que leur application en tant que médicaments.

L'état de la technique le plus proche est illustré par US-A 3 923 801 qui décrit des 5-alkyl-3-aryl-2H,5H-1,3-oxazino [5,6-c] 1,2-benzothiazine-2,4(3H)-dione 6,6-diosydes dont l'activité reste très inférieure à celle des dérivés de la présente invention.

Les nouveaux dérivés, objet de la présente invention, répondent à la formule générale I:

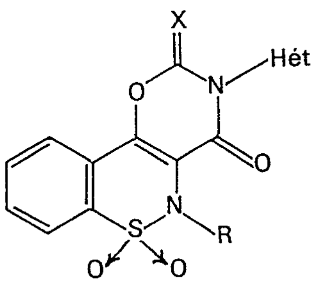

(I)

dans laquelle:

R représente un radical alcoyle inférieur linéaire ou ramifié en $C_1$ à $C_4$, en particulier les radicaux méthyle et éthyle,

X représente un atome d'oxygène ou de soufre Hét représente un hétérocycle insaturé mono- ou bicyclique contenant un hétéroatome d'azote en position ortho duquel ledit hétérocycle est relié à l'atome d'azote du cycle oxazinique, ledit hétérocycle contenant éventuellement 1 ou 2 autres hétéroatomes additionnels choisis parmi l'azote, l'oxygène et le soufre, et pouvant en outre être substitué par un ou deux radicaux méthyle.

Le radical hétérocyclique Hét entrant dans la formule générale I des dérivés de l'invention peut en particulier être constitué par un radical hétéromonocyclique insaturé comportant 5 ou 6 chaînons. A titre d'exemples particuliers d'un tel radical Hét on mentionnera les radicaux:

2-pyridyle; 2-(4-méthyl pyridyle); 2-(6-méthyl pyridyle); 2-pyrimidinyle; 5-méthyl-3-isoxazolyle; 2-thiazolyle; 4-méthyl-2-thiazolyle; 5-méthyl-2-thiazolyle; 4,5-diméthyl-2-thiazolyle; 3-isoxazolyle; 5-isoxazolyle; 5-méthyl-1,3,4-thiadiazolyle.

Le radical hétérocyclique Hét peut cependant également être constitué par un radical hétérobicyclique insaturé, tel que le radical 2-benzothiazolyle.

La présente invention concerne également un procédé pour la préparation des dérivés de formule générale I précédemment définie. Conformément à l'invention, on prépare les dérivés de formule générale I en faisant réagir un composé 2-alcoyl-4-hydroxy-2H-1,2-benzothiazine-1,1-dioxyde-3-carboxylate d'alcoyle de formule générale II:

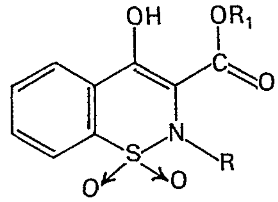

(II)

dans laquelle R et $R_1$ représentent indépendamment l'un de l'autre un radical alcoyle inférieur linéaire ou ramifié en $C_1$ à $C_4$, en particulier les radicaux méthyle et éthyle, avec un composé intermédiaire préparé in situ répondant à la formule générale III

$$[\text{Hét} - N = C = O] \qquad (III)$$

dans laquelle Hét a la signification donnée précédemment à propos de la formule générale I.

Cette réaction peut avoir lieu par fusion ou encore au sein de divers solvants inertes de point d'ébullition élevé, comme par exemple l'éther diméthylique du diéthylèneglycol et analogues, les hydrocarbures aromatiques comme le méthylnaphtalène ou d'autres hydrocarbures comme la tétraline ou la décaline.

Le composé intermédiaire de formule générale III peut en particulier être avantageusement préparé in situ dans le milieu réactionnel par décomposition thermique d'un dérivé d'uréthane de formule générale IV

$$\text{Hét} - NH - \overset{\overset{\displaystyle O}{\|}}{C} - O - R_2 \qquad (IV)$$

dans laquelle:

Hét a la signification précédemment donnée à propos de la formule générale I, et $R_2$ représente un radical phényle, éthyle, propyle, isopropyle, n-butyle, isobutyle ou tert-butyle. Les dérivés d'oxazinobenzothiazine-6,6-dioxyde de formule générale I dans laquelle X représente un atome de soufre se préparent à partir de dérivés d'oxazinobenzothiazine-6,6-dioxyde correspondants de formule générale I dans laquelle X représente un atome d'oxygène, par réaction avec un agent de thiation, c'est-à-dire susceptible de remplacer une double liaison $>C = O$ par une double liaison $>C = S$, par exemple choisi parmi $P_2S_5$ et le 2,4-bis-(4-méthoxyphényl)-2,4-dithioxo-1,3,2,4-dithiadiphosphétane.

Compte tenu de leur bonne activité anti-inflammatoire et analgésique et de leur très faible toxicité, les dérivés de formule générale I sont utiles en tant que médicaments utilisables en thérapeutique humaine ou animale. La présente invention se rapporte donc également à l'application de ces dérivés en tant que médicaments ainsi qu'aux compositions pharmaceutiques les contenant à titre de principe actif.

On décrira ci-après, à titre de simple exemple non limitatif, la préparation de quelques dérivés de formule générale I.

Exemple 1

Préparation du 5-éthyl-3-(2-pyridyl)-2H,5H-1,3-oxazino [5,6-c] [1,2] benzothiazine-2,4-(3H)dione-6,6-dioxyde

On chauffe au reflux à 161°C, sous atmosphère d'azote, pendant 30 minutes 4,4 g (0,015 mole) de 2-éthyle-4-hydroxy-2H-1,2-benzothiazine-1,1-dioxyde-3-carboxylate d'éthyle et 3,5 g (0,016 mole) de 2-phénoxycarbonylaminopyridine dans 40 ml d'éther diméthylique du diéthylèneglycol. On refroidit à température ambiante et on verse le contenu sur 150 ml d'eau. On filtre le précipité obtenu et on le lave à l'eau. Par recristallisation dans l'acétone on obtient 3,0 g (54%) de 5-éthyl-3-(2-pyridyl)-2H,5H-1,3-oxazino [5,6-c] [1,2] benzothiazine-2,4-(3H) dione-6,6-dioxyde de formule:

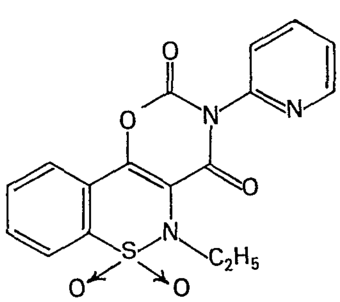

Point de fusion: 268–270°C

Données spectroscopiques:

IR (KBr): 1185; 1355; 1415; 1635; 1705; 1795 $cm^{-1}$ $^1H$ RMN, δ, [DMSO($d_6$)]: 1,08 (t,3H); 3,75 (q, 2H); 7,43 (é, 2H); 7,86 (é, 5H); 8,4 (d, 1H).

Exemple 2

Préparation de 5-méthyl-3-(2-pyridyl)-2H,5H-1,3-oxazino [5,6-c] [1,2] benzothiazine-2,4-(3H)dione-6,6-dioxyde

Variante A

On chauffe jusqu'à fusion dans un bain de silicone à 210°C, sous atmosphère d'azote, 14,1 g (0,05 mole) de 2-méthyl-4-hydroxy-2H-1,2-benzothiazine-1,1-dioxyde-3-carboxylate d'éthyle et 11,8 g (0,055 mole) de 2-phénoxycarbonylaminopyridine. On maintient la fusion pendant 10 minutes en distillant le phénol à pression réduite (25 mm de Hg). On dissout le résidu dans l'acétone d'où cristallisent 12,1 g (68%) de 5-méthyl-3-(2-pyridyl)-2H,5H-1,3-oxazino [5,6-c] [1,2] benzothiazine-2,4-(3H) dione-6,6-dioxyde, de formule:

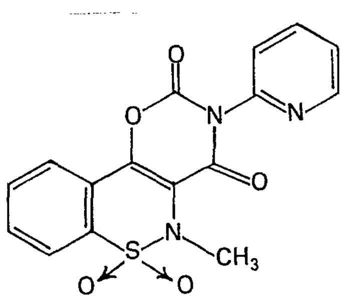

Point de fusion: 259–261°C

Données spectroscopiques:

IR (KBr): 1185; 1355; 1410; 1640; 1710; 1790 $cm^{-1}$ $^1H$ RMN, δ, [DMSO($d_6$)]: 3,02 (s, 3H); 7,52 (é, 2H); 7,92 (é, 5H); 8,52 (d, 1H).

Variante B

On chauffe à 200°C, pendant 15 minutes, en atmosphère d'azote, 2,7 (0,01 mole) de 2-méthyl-4-hydroxy-2H-1,2-benzothiazine-1,1-dioxyde-3-carboxylate de méthyle et 2,1 g (0,011 mole) de 2-t-butoxycarbonylaminopyridine dans 30 ml de 1-méthylnaphtalène. On refroidit et on précipite avec de l'éther de pétrole. Par recristallisation dans l'acétone on obtient 1,9 g (55%) de 5-méthyl-3-(2-pyridyl)-2H,5H-1,3-oxazino [5,6-c] [1,2] benzothiazine-2,4-(3H) dione-6,6-dioxyde, de point de fusion 257–259°C.

Données spectroscopiques:

IR (KBr): 1185; 1355; 1410; 1640; 1710; 1790 $cm^{-1}$ $^1H$ RMN, δ, [DMSO($d_6$)]: 3,02 (s, 3H); 7,52 (é, 2H); 7,92 (é, 5H); 8,52 (d, 1H).

Exemple 3

Préparation de 5-méthyl-3-(2-pyridyl)-2H,5H-1,3-oxazino [5,6-c] [1,2] benzothiazine-2-(3H)thione-4-one-6,6-dioxyde

On maintient au reflux à 140°C pendant 36 heures 8,1 g (0,02 mole) de 2,4-bis-(4-méthoxyphényl)-2,4-dithioxo-1,3,2,4-dithiadiphosphétane et 3,6 g (0,01 mole) de 5-méthyl-3-(2-pyridyl)-2H,5H-1,3-oxazino [5,6-c] [1,2] benzothiazine-2,4-(3H) dione-6,6-dioxyde dans 80 ml de xylène. On chromatographie sur colonne avec du gel de silice comme phase stationnaire en éluant d'abord avec du xylène et ensuite avec du chloroforme. On récupère de la solution chloroformique 1,4 g (38%) de 5-méthyl-3-(2-pyridyl)-2H,5H-1,3-oxazino [5,6-c] [1,2] benzothiazine-2 (3H) thione-4-one-6,6-dioxyde de formule:

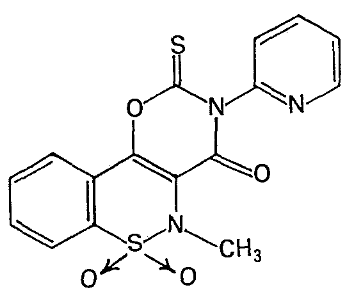

Après recristallisation dans l'acétone, le dérivé obtenu présente un point de fusion de 269–271°C.

Données spectroscopiques:

IR (KBr): 1190; 1320; 1355; 1400; 1640; 1712 $cm^{-1}$ $^1H$ RMN, δ, [DMSO($d_6$)]: 3,05 (s, 3H); 7,54 (é, 2H); 7,92 (é, 5H); 8,48 (d, 1H).

Exemple 4

Préparation de 5-méthyl-3-[3-(5-méthyl isoxazolyl)]-2H,5H-1,3-oxazino [5,6-c] [1,2] benzothiazine-2,4-(3H) dione-6,6-dioxyde

On chauffe à 200°C, pendant 15 minutes, sous atmosphère d'azote, 2,8 g (0,01 mole) de 2-méthyl-4-hydroxy-2H-1,2-benzothiazine-1,1-

dioxyde-3-carboxylate d'éthyle et 2,4 g (0,011 mole) de 3-phénoxy-carbonylamino-5-méthyl-isoxazole dans 40 ml de 1-méthylnaphtalène. On refroidit et on précipite avec de l'éther de pétrole. On recristallise dans l'acétone et on obtient 2,2 g (61%) de 5-méthyl-3-[3-(5-méthyl-isoxazolyl)]-2H,5H-1,3-oxazino [5,6-c] [1,2] benzothiazine-2,4-(3H) dione-6,6-dioxyde de formule:

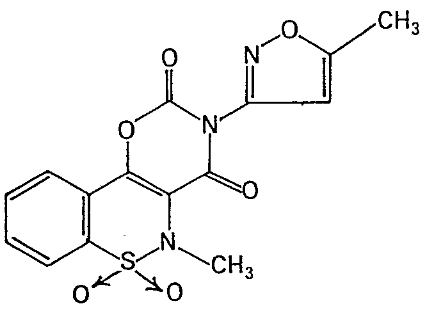

Point de fusion 268–269°C

Données spectroscopiques:

IR (KBr): 1185; 1360; 1400; 1640; 1715; 1788 $cm^{-1}$ $^1H$ RMN, δ, [DMSO($d_6$)]: 2,5 (s, 3H); 3,08 (s, 3H); 6,44 (s, 1H); 7,98 (é, 4H).

Exemple 5

Préparation de 5-méthyl-3-(2-pyrimidinyl)-2H, 5H-1,3-oxazino [5,6-c] [1,2] benzothiazine-2,4-(3H) dione-6,6-dioxyde

On chauffe au reflux à 161°C, pendant 30 minutes, sous atmosphère d'azote, 2,8 g (0,01 mole) de 2-méthyl-4-hydroxy-2H-1,2-benzothiazine-1,1-dioxyde-3-carboxylate d'éthyle et 2,4 g (0,011 mole) de 2-phénoxycarbonyl-aminopyrimidine dans 30 ml d'éther diméthylique du diéthylèneglycol. On refroidit à température ambiante et on verse sur 100 ml d'eau. On filtre le précipité obtenu, on lave à l'eau, on agite avec de l'acétone à ébullition et on filtre à nouveau. On obtient 1,8 g (50%) de 5-méthyl-3-(2-pyrimidinyl)-2H,5H-1,3-oxazino [5,6-c] [1,2] benzothiazine-2,4-(3H) dione 6,6-dioxyde de formule:

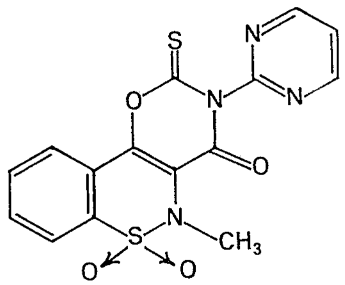

Point de fusion 282–284°C

Données spectroscopiques:

IR (KBr): 1180; 1355; 1400; 1632; 1710; 1787 $cm^{-1}$ $^1H$ RMN, δ, [DMSO($d_6$)]: 3,05 (s, 3H); 7,93 (é, 5H); 8,95 (d, 2H).

Exemple 6

Préparation de 5-méthyl-3-[2-(4-méthyl pyridyl)]-2H,5H-1,3-oxazino [5,6-c] [1,2] benzothiazine-2,4-(3H) dione-6,6-dioxyde

On chauffe jusqu'à fusion dans un bain de silicone à 160°C, sous atmosphère d'azote, 2,7 g (0,01 mole) de 2-méthyl-4-hydroxy-2H-1,2-benzothiazine-1,1-dioxyde-3-carboxylate de méthyle et 2,3 g (0,011 mole) de 2-t-butoxycarbonylamino-4-méthylpyridine. On maintient la fusion pendant 5 minutes. On dissout le résidu dans l'acétone, d'où cristallisent 2,3 g (62%) de 5-méthyl-3-[2-(4-méthyl pyridyl)]-2H,5H-1,3-oxazino [5,6-c] [1,2] benzothiazine-2,4-(3H) dione-6,6-dioxyde, de formule:

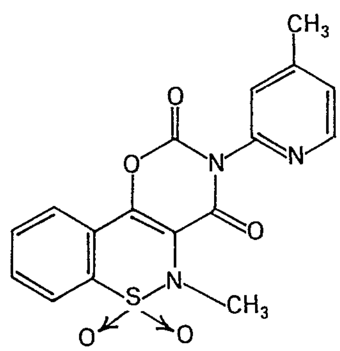

Point de fusion 259–261°C

Données spectroscopiques:

IR (KBr): 1195; 1360; 1415; 1640; 1720; 1795 $cm^{-1}$ $^1H$ RMN, δ, [DMSO($d_6$)]: 2,42 (s, 3H); 3,1 (s, 3H); 7,4 (é, 2H); 7,98 (é, 4H); 8,4 (d, 1H).

Exemple 7

Préparation de 5-méthyl-3-[2-(6-méthyl pyridyl)]-2H,5H-1,3-oxazino [5,6-c] [1,2] benzothiazine-2,4-(3H) dione-6,6-dioxyde

On chauffe jusqu'à fusion dans un bain de silicone à 160°C, sous atmosphère d'azote, 2,8 g (0,01 mole) de 2-méthyl-4-hydroxy-2H-1,2-benzothiazine-1,1-dioxyde-3-carboxylate d'éthyle et 2,3 g (0,011 mole) de 2-t-butoxycarbonylamino-6-méthyl-pyridine. On maintient la fusion pendant 5 minutes. On dissout le résidu dans l'acétone d'où cristallisent 1,5 g (41%) de 5-méthyl-3-[2-(6-méthyl pyridyl)]-2H,5H-1,3-oxazino [5,6-c] [1,2] benzothiazine-2,4-(3H) dione-6,6-dioxyde de formule:

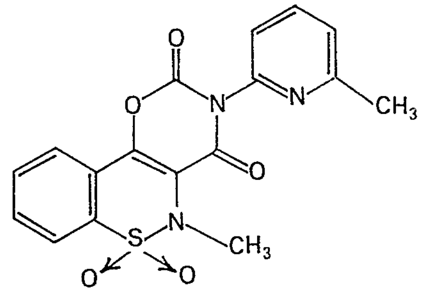

Point de fusion 248–250°C

Données spectroscopiques:

IR (KBr): 1190; 1360; 1410; 1640; 1715;

1790 $cm^{-1}$ $^1H$ RMN, δ, [DMSO($d_6$)]: 2,43 (s, 3H); 3,0 (s, 3H); 7,4 (é, 2H); 7,97 (é, 5H).

Activité anti-inflammatoire (inhibition de l'œdème produit par la carraghénine)

Méthode de Winter (Winter, C.A., Risley, E.A. et Nuss, G.W. Proc. Soc. Exp. Biol. Med., 11, 544–540 [1962]).

On utilise des rats mâles de souche Sprague-Dawley HC/CEY d'un poids compris entre 90 et 110 g. Les produits sont administrés en suspension dans de la gomme arabique à 5%, par voie orale, moyennant une sonde œsophagique, 1 heure avant l'injection subplantaire dans la patte droite de chaque animal de 0,1 ml de suspension de carraghénine à 1% dans du sérum physiologique (NaC1 0,9%). Les animaux témoins reçoivent la gomme arabique à 5%, le volume total employé dans tous les cas étant de 10 ml/kg.

On mesure le volume de chaque patte immédiatement avant l'injection de carraghénine (temps 0), puis trois et cinq heures après celle-ci, au moyen d'un pléthysmographe à mercure.

On calcule pour chaque animal l'indice d'inflammation au bout de 3 heures ($I_3$) et au bout de 5 heures ($I_5$) après l'injection de carraghénine moyennant les formules suivantes:

$$I_3 = \frac{V_3}{V_0} \times 100 \qquad I_5 = \frac{V_5}{V_0} \times 100$$

dans lesquelles:

$V_0$ = volume de la patte au temps zéro (immédiatement avant l'administration de carraghénine)

$V_3$ = volume de la patte 3 heures après l'injection de carraghénine

$V_5$ = volume de la patte 5 heures après l'injection de carraghénine.

On calcule l'indice d'inflammation moyen pour le lot témoin au bout de 3 heures ($IT_3$) et au bout de 5 heures ($IT_5$).

Le pourcentage d'inhibition de l'œdème (activité anti-inflammatoire) est calculé pour chaque animal moyennant les formules:

$$A_3 = \frac{(It_3 - I_3)}{(It_3 - 100)} \times 100 \qquad A_5 = \frac{(It_5 - I_5)}{(It_5 - 100)} \times 100$$

dans lesquelles:

$A_3$ = activité anti-inflammatoire en pourcentage d'inhibition de l'œdème au bout de 3 heures

$A_5$ = activité anti-inflammatoire en pourcentage d'inhibition de l'œdème au bout de 5 heures.

L'activité anti-inflammatoire est proportionnelle au logarithme de la dose administrée. A partir de la courbe logarithme dose – effet %, on obtient la dose qui réduit le volume de l'œdème de 25%. On adopte cette dose comme DE-50, puisque l'effet maximum atteint est la réduction de l'œdème de 50%.

Le tableau I ci-après récapitule les résultats obtenus avec les dérivés des exemples précédemment décrits.

Tableau 1

| Produit | DE-50 en mg/kg p.o. 3 heures | 5 heures |
|---|---|---|
| Exemple 1 | > 40 | > 40 |
| Exemple 2 | 2,3 | 2,6 |
| Exemple 3 | > 40 | > 40 |
| Exemple 4 | 38 | 39 |
| Exemple 5 | > 40 | > 40 |
| Exemple 6 | > 40 | > 40 |
| Exemple 7 | 20 | 25 |
| Phénylbutazone | 17 | 17 |

Activité analgésique face à la douleur induite par l'acétylcholine

Méthode de Collier (Collier, H.O.W. Dinneen, L.C. Johnson, C.A. et Schneider, C.-Br. J. Pharmac. Chemother., 32, 295–310 [1968]).

On utilise des souris mâles de souche Swiss d'un poids compris entre 20 et 25 g. On place les souris dans des cages individuelles. On administre à chaque animal, par voie intrapéritonéale, un volume de 0,2 ml/20 g d'une solution de bromure d'acétylcholine de 0,32 mg/ml. La dose de bromure d'acétylcholine injectée est donc de 3,2 mg/kg. On compte, pendant 5 minutes, le nombre de contorsions ou d'étirements qu'effectue chaque souris. Après 5 minutes on administre les produits en employant comme véhicule la gomme arabique à 5%. Au bout de 40 et de 120 minutes de l'administration des produits, on redonne une injection d'acétylcholine et on compte les contorsions observées pendant 5 minutes.

On calcule pour chaque animal, le pourcentage d'inhibition du nombre de contorsions au bout de 40 et de 120 minutes moyennant les formules suivantes:

$$A_{40} = \left( \frac{N_0 - N_{40}}{N_0} \right) \times 100 \qquad A_{120} = \left( \frac{N_0 - N_{120}}{N_0} \right) \times 100$$

dans lesquelles:

$N_0$ = nombre de contorsions avant l'administration du produit

$N_{40}$ = nombre de contorsions 40 minutes après l'administration du produit

$N_{120}$ = nombre de contorsions 120 minutes après l'administration du produit.

Le pourcentage d'inhibition des contorsions est directement proportionnel au logarithme de la dose administrée. A partir de la courbe logarithme dose – effet % on obtient la dose qui réduit le nombre de contorsions jusqu'à la moitié de la valeur initiale (DE-50).

Le tableau II ci-après récapitule les résultats obtenus avec les dérivés des exemples précédemment décrits.

Tableau 2

| Produit | DE-50 mg/kg p.o 40 min. | 120 min. |
|---|---|---|
| Exemple 1 | > 160 | > 160 |
| Exemple 2 | 1,8 | 1,1 |
| Exemple 3 | 25 | 25 |
| Exemple 4 | 154 | 154 |
| Exemple 5 | > 160 | > 160 |
| Exemple 6 | > 160 | > 160 |
| Exemple 7 | 7,5 | 10 |
| Phénylbutazone | 40 | 35 |

Inhibition de la prostaglandine-synthétase de vésicule séminale de bœuf

Méthode de Yoshimoto (Yoshimoto, A., Ito, H. et Tomita, K.-J. Biochem., 68, 487 [1970].

La détermination de l'activité de la prostaglandine-synthétase (1.14.99.1) s'effectue en mesurant les augmentations d'absorbance à 278 nm dues à la formation de la prostaglandine B à partir de la prostaglandine E, en milieu alcalin.

La température d'incubation a été dans tous les cas de 37 ± 0,1 °C. Le procédé utilisé est mis en œuvre comme suit:

à 1,5 ml de solution de cofacteur (0,55 mg d'hydroquinone + 0,5 mg d'hémoglobine + 7,7 mg de glutathion dans 12 ml de tampon Tris-HCl 0,2 M de pH 8,0), contenant 10 mg d'enzyme, on ajoute 0,5 ml de solution de substrat (0,6 mg d'acide araquidonique dans 10 ml de tampon) et 0,5 ml de tampon, et on effectue une incubation à 37°C pendant 10 minutes. On arrête la réaction enzymatique par addition de 1,5 ml d'acide citrique 0,2 M et la pGE s'extrait avec 2×5 ml d'acétate d'éthyle. On évapore la phase organique sous courant d'azote à 40°C et le résidu est redissous dans 2 ml de méthanol et 0,5 ml d'hydroxyde de potassium 3 M. Finalement on mesure les absorptions à 278 nm face à un blanc auquel on ajoute 0,5 ml de tampon au lieu de 0,5 ml de solution de substrat.

On exprime l'activité enzymatique comme augmentation de la densité optique pendant 10 minutes de réaction.

L'inhibition exercée par la phénylbutazone et par exemple le dérivé correspondant à l'exemple 2 sur la prostaglandine-synthétase, se détermine en maintenant l'enzyme en contact avec les produits pendant 5 minutes. La méthode est la suivante:

à 1,5 ml de solution de cofacteur, contenant 10 mg d'enzyme, on ajoute 0,5 ml des différentes solutions de phénylbutazone ou du dérivé de l'exemple 2, et on maintient à 37°C pendant 5 minutes, après lesquelles on ajoute 0,5 ml de solution de substrat et on maintient l'incubation pendant 10 minutes supplémentaires à la même température.

Les étapes postérieures sont identiques à celles décrites antérieurement. La relation entre l'augmentation de densité optique des essais avec et sans inhibiteur est la valeur du pourcentage d'inhibition.

Dans le tableau III on indique, pour la phénylbutazone et le dérivé de l'exemple 2, les concentrations qui produisent une inhibition de 50%.

Tableau 3
Inhibition de la prostaglandine-synthétase (1.14.99.1) par la phénylbutazone et le dérivé de l'exemple 2, in vitro.

| Produit | $CI_{50}$(M) |
|---|---|
| Phénylbutazone | $3 \times 10^{-4}$ |
| Exemple 2 | $1 \times 10^{-4}$ |

Toxicité aigüe

Méthode de Litchfield et Wilcoxon (Litchfield, J.T. et Wilcoxon, E.-J. Pharmacol. Exp. Therap., 96, 19-113 [1949]).

On administre le produit par voie orale en suspension dans la gomme arabique à 5%. Le volume administré est de 25 ml/kg chez la souris et de 10 ml/kg chez le rat. On calcule selon la méthode citée la DL-50. Les résultats obtenus pour le dérivé de l'exemple 2 sont mentionnés dans le tableau IV ci-après.

Tableau 4

| Espèce | Sexe | DL-50 (mg/kg) v.o. |
|---|---|---|
| Souris | ♂ | 6 192 |
| | ♀ | 8 841 |
| Rat | ♂ | 1 434 |
| | ♀ | 1 994 |

Compte tenu de leurs bonnes propriétés pharmacodynamiques, les dérivés d'oxazinobenzothiazine-6,6-dioxyde selon l'invention peuvent être utilisés de façon satisfaisante en thérapeutique humaine et animale, en particulier dans le traitement des affections aigües ou chroniques qui nécessitent l'utilisation d'agents anti-inflammatoires et/ou analgésiques, telles que par exemple l'arthrite déformante, l'ostéoarthrite, la spondylite, les altérations musculo-squelettiques aigües et la goutte aigüe.

En thérapeutique humaine, la dose d'administration des dérivés de la présente invention est bien sûr fonction de la gravité de l'affection à traiter. Elle sera généralement comprise entre environ 40 et environ 100 mg/jour. Les dérivés de l'invention seront par exemple administrés sous la forme de comprimés, de gélules ou de suppositoires.

On indiquera ci-après, à titre d'exemples, trois formes galéniques particulières des dérivés objet de la présente invention.

Exemple de formule par gélule

| | |
|---|---|
| 5-méthyl-3-(2-pyridyl)-2H,5H-1,3-oxazino [5,6-c] [1,2] benzothiazine-2,4-(3H) dione-6,6-dioxyde | 0,020 g |
| Lactose | 0,136 g |

| | |
|---|---|
| Talc | 0,0016 g |
| Stéarate de magnésium | 0,0016 g |
| Aerosil-200 | 0,0008 g |
| Poids gélule | 0,160 g |

Exemple de formule par comprimé

| | |
|---|---|
| 5-méthyl-3-(2-pyridyl)-2H,5H-1,3-oxazino [5,6-c] [1,2] benzothiazine-2,4-(3H) dione-6,6-dioxyde | 0,020 g |
| Avicel pH 102 | 0,016 g |
| Lactose | 0,055 g |
| Primojel | 0,003 g |
| Polivinylpyrrolidone | 0,005 g |
| Stéarate de magnésium | 0,001 g |
| Poids comprimé | 0,100 g |

Exemple de formule par suppositoire

| | |
|---|---|
| 5-méthyl-3-(2-pyridyl)-2H,5H-1,3-oxazino [5,6-c] [1,2] benzothiazine-2,4-(3H) dione-6,6-dioxyde | 0,050 g |
| Monolène | 1,950 g |
| Poids suppositoire | 2,000 g |

**Revendications pour les Etats contractants: BE, CH, DE, FR, GB, IT, LI, LU, NL, SE**

1. Nouveaux dérivés d'oxazinobenzothiazine-6,6-dioxyde répondant à la formule générale I:

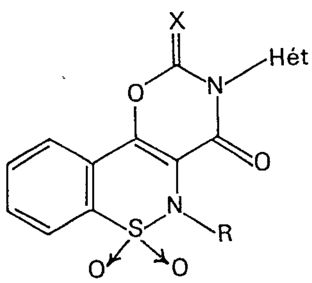
(I)

dans laquelle:

R représente un radical alcoyle inférieur linéaire ou ramifié en $C_1$ à $C_4$, en particulier les radicaux méthyle et éthyle,

X représente un atome d'oxygène ou de soufre Hét représente un hétérocycle insaturé mono- ou bicyclique contenant un hétéroatome d'azote en position ortho duquel ledit hétérocycle est relié à l'atome d'azote du cycle oxazinique, ledit hétérocycle contenant éventuellement 1 ou 2 autres hétéroatomes additionnels choisis parmi l'azote, l'oxygène et le soufre, et pouvant en outre être substitué par un ou deux radicaux méthyle.

2. Nouveaux dérivés de formule générale I selon la revendication 1, caractérisés en ce que Hét représente un radical hétéromonocyclique insaturé à 5 ou 6 chaînons.

3. Nouveaux dérivés de formule générale I selon l'une des revendications 1 ou 2, caractérisés en ce que Hét représente un radical hétérocyclique choisi parmi les radicaux suivants:

2-pyridyle; 2-(4-méthyl pyridyle); 2-(6-méthyl pyridyle); 2-pyrimidinyle; 5-méthyl-3-isoxazolyle; 2-thiazolyle; 4-méthyl-2-thiazolyle; 5-méthyl-2-thiazolyle; 4,5-diméthyl-2-thiazolyle; 2-benzothiazolyle; 3-isoxazolyle; 5-isoxazolyle; 5-méthyl-1,3,4-thiadiazolyle.

4. Nouveaux dérivés de formule générale I selon l'une des revendications 1 à 3, caractérisés en ce qu'ils sont choisis parmi:

- le 5-éthyl-3-(2-pyridyl)-2H,5H,-1,3-oxazino[5,6-c] [1,2] benzothiazine-2,4-(3H)dione-6,6-dioxyde;
- le 5-méthyl-3-(2-pyridyl)-2H,5H-1,3-oxazino[5,6-c] [1,2] benzothiazine-2,4-(3H) dione-6,6-dioxyde;
- le 5-méthyl-3-(2-pyridyl)-2H,5H-1,3-oxazino[5,6-c] [1,2] benzothiazine-2-(3H)thione-4-one-6,6-dioxyde;
- le 5-méthyl-3-[3-(5-méthyl isoxazolyl)]-2H,5H-1,3-oxazino[5,6-c] [1,2] benzothiazine-2,4-(3H) dione-6,6-dioxyde;
- le 5-méthyl-3-(2-pyrimidinyl)-2H,5H-1,3-oxazino[5,6-c] [1,2] benzothiazine-2,4-(3H) dione-6,6-dioxyde;
- le 5-méthyl-3-[2-(4-méthyl pyridyl)]-2H,5H-1,3-oxazino[5,6-c] [1,2] benzothiazine-2,4-(3H) dione-6,6-dioxyde, et
- le 5-méthyl-3-[2-(6-méthyl pyridyl)]-2H,5H-1,3-oxazino[5,6-c] [1,2] benzothiazine-2,4-(3H) dione-6,6-dioxyde.

5. Procédé de préparation des dérivés de formule générale I selon l'une des revendications 1 à 4, caractérisé en ce que l'on fait réagir un composé de formule générale II

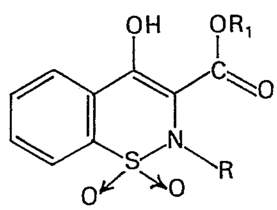
(II)

dans laquelle R et $R_1$ représentent indépendamment l'un de l'autre un radical alcoyle inférieur linéaire ou ramifié en $C_1$ à $C_4$, en particulier les radicaux méthyle et éthyle, avec un composé intermédiaire préparé in situ répondant à la formule générale III

$$[Hét - N = C = O] \quad (III)$$

dans laquelle Hét a la signification donnée dans l'une des revendications 1 à 3 à propos de la formule générale I.

6. Procédé selon la revendication 5, caractérisé en ce que le composé intermédiaire de formule générale III est préparé in situ par décomposition thermique d'un dérivé d'uréthane de formule générale IV

$$Hét - NH - \overset{O}{\overset{\|}{C}} - O - R_2 \quad (IV)$$

dans laquelle:

Hét a la signification donnée dans l'une des revendications 1à 3 à propos de la formule générale I, et

$R_2$ représente un radical phényle, éthyle, propyle, isopropyle, n-butyle, isobutyle ou tert-butyle.

7. Procédé selon l'une des revendications 5 et 6, caractérisé en ce que, pour obtenir un dérivé de formule générale I dans laquelle X représente un atome de soufre, on fait réagir un composé de formule générale I dans laquelle X représente un atome d'oxygène avec un agent de thiation.

8. Procédé selon la revendication 7, caractérisé en ce que l'agent de thiation est choisi parmi $P_2S_5$ et le 2,4-bis-(4-méthoxyphényl)-2,4-dithioxo 1,3,2,4-dithiadiphosphétane.

9. A titre de médicaments nouveaux les dérivés de formule générale I selon l'une des revendications 1 à 4.

10. Compositions pharmaceutiques caractérisées en ce qu'elles contiennent, outre un support pharmaceutiquement acceptable, un principe actif comprenant au moins un dérivé de formule générale I selon l'une des revendications 1 à 4.

**Revendications pour l'Etat contractant: AT**

1. Procédé de préparation des dérivés d'oxazinobenzothiazine-6,6-dioxyde répondant à la formule générale I:

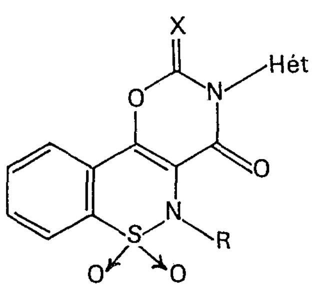

(I)

dans laquelle:

R représente un radical alcoyle inférieur linéaire ou ramifié en $C_1$ à $C_4$, en particulier les radicaux méthyle et éthyle,

X représente un atome d'oxygène ou de soufre,

Hét représente un hétérocycle insaturé mono- ou bicyclique contenant un hétéroatome d'azote en position ortho duquel ledit hétérocycle est relié à l'atome d'azote du cycle oxazinique, ledit hétérocycle contenant éventuellement 1 ou 2 autres hétéroatomes additionnels choisis parmi l'azote, l'oxygène et le soufre, et pouvant en outre être substitué par un ou deux radicaux méthyle,

caractérisé en ce que l'on fait réagir un composé de formule générale II:

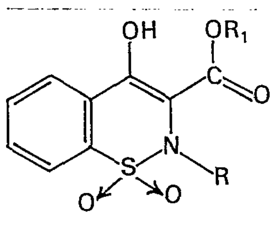

(II)

dans laquelle R et $R_1$ représentent indépendamment l'un de l'autre un radical alcoyle inférieur linéaire ou ramifié en $C_1$ à $C_4$, en particulier les radicaux méthyle et éthyle, avec un composé intermédiaire prépré in situ répondant à la formule générale III:

$$[\text{Hét} - N = C = O] \qquad (III)$$

dans laquelle Hét a la signification donnée précédemment.

2. Procédé selon la revendication 1, caractérisé en ce que le composé intermédiaire de formule générale III est préparé in situ par décomposition thermique d'un dérivé d'uréthane de formule générale IV:

$$\text{Hét} - NH - \overset{\overset{\displaystyle O}{\|}}{C} - O - R_2 \qquad (IV)$$

dans laquelle:

Hét a la signification donnée à la revendication 1, à propos de la formule générale I, et

$R_2$ représente un radical phényle, éthyle, propyle, isopropyle, n-butyle, isobutyle ou tert-butyle.

3. Procédé selon l'une des revendications 1 et 2, caractérisé en ce que, pour obtenir un dérivé de formule générale I dans laquelle X représente un atome de soufre, on fait réagir un composé de formule générale I dans laquelle X représente un atome d'oxygène avec un agent de thiation.

4. Procédé selon la revendication 3, caractérisé en ce que l'agent de thiation est choisi parmi $P_2S_5$ et le 2,4-bis-(4-méthoxyphényl)-2,4-dithioxo 1,3,2,4-dithiadiphosphétane.

5. Procédé selon la revendication 1, caractérisé en ce que l'on prepare un composé de formule générale I dans laquelle Hét représente un radical hétéromonocyclique insaturé à 5 ou 6 chaînons.

6. Procédé selon la revendication 1, caractérisé en ce que l'on prépare un composé de formule générale I dans laquelle Hét représente un radical hétérocyclique choisi parmi les radicaux suivants:

2-pyridyle; 2-(4-méthyl pyridyle); 2-(6-méthyl pyridyle); 2-pyrimidinyle; 5-méthyl-3-isoxazolyle; 2-thiazolyle; 4-méthyl-2-thiazolyle; 5-méthyl-2-thiazolyle; 4,5-diméthyl-2-thiazolyle; 2-benzothiazolyle; 3-isoxazolyle; 5-isoxazolyle; 5-méthyl-1,3,4-thiadiazolyle.

7. Procédé selon la revendication 1, caractérisé en ce que l'on prépare un composé de formule générale I choisi parmi:

- le 5-éthyl-3-(2-pyridyl)-2H,5H-1,3-oxazino[5,6-c] [1,2] benzothiazine-2,4-(3H)dione-6,6-dioxyde;
- le 5-méthyl-3-(2-pyridyl)-2H,5H-1,3-oxazino[5,6-c] [1,2] benzothiazine-2,4-(3H) dione-6,6-dioxyde;
- le 5-méthyl-3-(2-pyridyl)-2H,5H-1,3-oxazino[5,6-c] [1,2] benzothiazine-2-(3H)thione-4-one-6,6-dioxyde;
- le 5-méthyl-3-[3-(5-méthyl isoxazolyl)]-2H,5H-1,3-oxazino[5,6-c] [1,2] benzothiazine-2,4-(3H) dione-6,6-dioxyde;

- le 5-méthyl-3-(2-pyrimidinyl)-2H,5H-1,3-oxazino[5,6-c] [1,2] benzothiazine-2,4-(3H) dione-6,6-dioxyde;
- le 5-méthyl-3-[2-(4-méthyl pyridyl)]-2H,5H-1,3-oxazino [5,6-c] [1,2] benzothiazine-2,4-(3H) dione-6,6-dioxyde, et
- le 5-méthyl-3-[2-(6-méthyl pyridyl)]-2H,5H-1,3-oxazino [5,6-c] [1,2] benzothiazine-2,4-(3H) dione-6,6-dioxyde.

**Patentansprüche für die Vertragsstaaten: BE, CH, DE, FR, GB, IT, LI, LU, NL, SE**

1. Neue Oxazinobenzothiazin-6,6-dioxid-derivate der allgemeinen Formel (I)

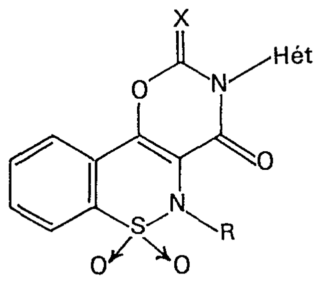
(I)

in welcher

R eine verzweigte oder geradkettige Alkylgruppe mit 1 bis 4 C-Atomen, insbesondere die Methyl- oder Äthylgruppe, darstellt,

X ein Sauerstoffatom oder ein Schwefelatom bedeutet und Het eine mono- oder bicyclische ungesättigte heterocyclische Gruppe mit einem Stickstoffatom als Heteroatom in Orthostellung zur Stellung mit welcher die heterocyclische Gruppe an das Stickstoffatom des Oxazinringes gebunden ist, bedeutet, wobei diese heterocyclische Gruppe gegebenenfalls 1 oder 2 weitere Heteroatome aus der von Stickstoff, Sauerstoff und Schwefel gebildeten Gruppe enthält und weiter durch eine oder zwei Methylgruppen substituiert sein kann.

2. Neue Derivate der allgemeinen Formel (I) nach Anspruch 1, dadurch gekennzeichnet, dass Het für eine 5- oder 6gliedrige monocyclische ungesättigte heterocyclische Gruppe steht.

3. Neue Derivate der allgemeinen Formel (I) nach einem der Ansprüche 1 oder 2, dadurch gekennzeichnet, dass Het für eine heterocyclische Gruppe steht, die unter den folgenden Gruppen

2-Pyridyl, 2-(4-Methyl-pyridyl), 2-(6-Methyl-pyridyl), 2-Pyrimidinyl, 5-Methyl-3-isoxazolyl, 2-Thiazolyl, 4-Methyl-2-thiazolyl, 5-Methyl-2-thiazolyl, 4,5-Dimethyl-2-thiazolyl, 2-Benzothiazolyl, 3-Isoxazolyl, 5-Isoxazolyl und 5-Methyl-1,3,4-thiadiazolyl ausgewählt ist.

4. Neue Derivate der allgemeinen Formel (I) nach einem der Ansprüche 1 bis 3, dadurch gekennzeichnet, dass sie unter

5-Äthyl-3-(2-pyridyl)-2H,5H-1,3-oxazino[5,6-c] [1,2]benzothiazin-2,4-(3H)dion-6,6-dioxid,

5-Methyl-3-(2-pyridyl)-2H,5H-1,3-oxazino[5,6-c] [1,2]benzothiazin-2,4-(3H)dion-6,6-dioxid,

5-Methyl-3-(2-pyridyl)-2H,5H-1,3-oxazino[5,6-c] [1,2]benzothiazin-2-(3H)thion-4-on-6,6-dioxid,

5-Methyl-3-[3-(5-methyl-isoxazolyl)]-2H,5H-1,3-oxazino[5,6-c] [1,2]benzothiazin-2,4-(3H)dion-6,6-dioxid,

5-Methyl-3-(2-pyrimidinyl)-2H,5H-1,3-oxazino[5,6-c] [1,2]benzothiazin-2,4-(3H)dion 6,6-dioxid,

5-Methyl-3-[2-(4-methyl-pyridyl)]-2H,5H-1,3-oxazino[5,6-c] [1,2]benzothiazin-2,4-(3H)dion-6,6-dioxid und

5-Methyl-3-[2-(6-methyl-pyridyl)]-2H,5H-1,3-oxazino[5,6-c] [1,2]benzothiazin-2,4-(3H)dion-6,6-dioxid ausgewählt sind.

5. Verfahren zum Herstellen von Derivaten der allgemeinen Formel (I) nach einem der Ansprüche 1 bis 4, dadurch gekennzeichnet, dass man eine Verbindung der allgemeinen Formel (II)

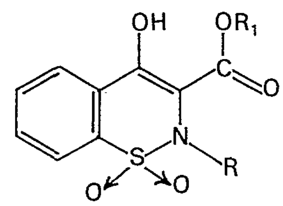
(II)

in welcher R und $R_1$ unabhängig voneinander eine geradkettige oder verzweigtkettige niedere Alkylgruppe mit 1 bis 4 Kohlenstoffatomen, insbesondere die Methyl- und Äthylgruppe, bedeuten, mit einem in situ hergestellten Zwischenprodukt der allgemeinen Formel (III)

$$[Het - N = C = O] \qquad (III)$$

in welcher Het die in einem der Ansprüche 1 bis 3 für die allgemeine Formel (I) angegebene Bedeutung besitzt, umsetzt.

6. Verfahren nach Anspruch 5, dadurch gekennzeichnet, dass das Zwischenprodukt der allgemeinen Formel (III) in situ durch thermische Zersetzung eines Urethanderivates der allgemeinen Formel (IV)

$$\text{Hét} - NH - \overset{\overset{\displaystyle O}{\|}}{C} - O - R_2 \qquad (IV)$$

hergestellt wird, in welcher

Het die in einem der Ansprüche 1 bis 3 für die allgemeine Formel (I) angegebene Bedeutung besitzt und

$R_2$ eine Phenyl-, Äthyl-, Propyl-, Isopropyl-, n-Butyl-, Isobutyl- oder t-Butylgruppe bedeutet.

7. Verfahren nach einem der Ansprüche 5 oder 6, dadurch gekennzeichnet, dass zwecks Herstellung eines Derivats der allgemeinen Formel (I) mit der Bedeutung eines Schwefelatoms für X eine Verbindung der allgemeinen Formel (I) mit der Bedeutung eines Sauerstoffatoms für X mit einem Schwefelungsmittel umgesetzt wird.

8. Verfahren nach Anspruch 7, dadurch gekennzeichnet, dass als Schwefelungsmittel $P_2S_5$ oder 2,4-Bis-(4-methoxyphenyl)-2,4-dithioxo-1,3,2,4-dithiadiphosphäthan eingesetzt wird.

9. Als neue Heilmittel, Derivate der allgemeinen Formel (I) nach einem der Ansprüche 1 bis 4.

10. Pharmazeutische Mischungen, dadurch gekennzeichnet, dass sie ausser einem pharmazeutisch annehmbaren Träger einen Wirkstoff enthalten, welcher von zumindest einem Derivat der allgemeinen Formel (I) nach einem der Ansprüche 1 bis 4 gebildet ist.

**Patentansprüche für den Vertragsstaat: AT**

1. Verfahren zum Herstellen von Oxazinobenzothiazin-6,6-dioxid-derivaten der allgemeinen Formel (I)

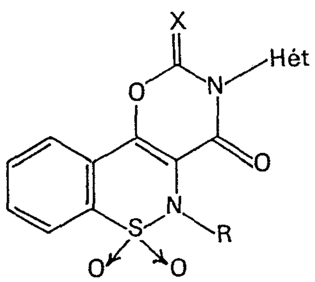

(I)

in welcher

R eine verzweigte oder geradkettige Alkylgruppe mit 1 bis 4 C-Atomen, insbesondere die Methyl- oder Äthylgruppe, darstellt,

X ein Sauerstoffatom oder ein Schwefelatom bedeutet und

Het eine mono- oder bicyclische ungesättigte heterocyclische Gruppe mit einem Stickstoffatom als Heteroatom in Orthostellung zur Stellung, mit welcher die heterocyclische Gruppe an das Stickstoffatom des Oxazinringes gebunden ist, bedeutet, wobei diese heterocyclische Gruppe gegebenenfalls 1 oder 2 weitere Heteroatome aus der von Stickstoff, Sauerstoff und Schwefel gebildeten Gruppe enthält und weiter durch eine oder zwei Methylgruppen substituiert sein kann, dadurch gekennzeichnet, dass man eine Verbindung der allgemeinen Formel (II)

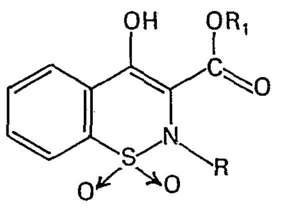

(II)

in welcher R und $R_1$ unabhängig voneinander eine geradkettige oder verzweigtkettige niedere Alkylgruppe mit 1 bis 4 Kohlenstoffatomen, insbesondere die Methyl- und Äthylgruppe, bedeuten, mit einem in situ hergestellten Zwischenprodukt der allgemeinen Formel (III)

[Het - N = C = O] (III)

in welcher Het die oben angegebene Bedeutung besitzt, umsetzt.

2. Verfahren nach Anspruch 1, dadurch gekennzeichnet, dass das Zwischenprodukt der allgemeinen Formel (III) in situ durch thermische Zersetzung eines Urethanderivates der allgemeinen Formel (IV)

O
||
Hét - NH - C - O - $R_2$ (IV)

hergestellt wird, in welcher

Het die in Anspruch 1 für die allgemeine Formel (I) angegebene Bedeutung besitzt und $R_2$ eine Phenyl-, Äthyl-, Propyl-, Isopropyl-, n-Butyl-, Isobutyl- oder t-Butylgruppe bedeutet.

3. Verfahren nach einem der Ansprüche 1 und 2, dadurch gekennzeichnet, dass zwecks Herstellung eines Derivats der allgemeinen Formel (I) mit der Bedeutung eines Schwefelatoms für X eine Verbindung der allgemeinen Formel (I) mit der Bedeutung eines Sauerstoffatoms für X mit einem Schwefelungsmittel umgesetzt wird.

4. Verfahren nach Anspruch 3, dadurch gekennzeichnet, dass als Schwefelungsmittel $P_2S_5$ oder 2,4-Bis-(4-methoxyphenyl)-2,4-dithioxo-1,3,2,4-dithiadiphosphäthan eingesetzt wir.

5. Verfahren nach Anspruch 1, dadurch gekennzeichnet, dass eine Verbindung der allgemeinen Formel (I) hergestellt wird, in welcher Het für eine 5- oder 6gliedrige monocyclische ungesättigte heterocyclische Gruppe steht.

6. Verfahren nach Anspruch 1, dadurch gekennzeichnet, dass eine Verbindung der allgemeinen Formel (I) hergestellt wird, in welcher Het für eine heterocyclische Gruppe steht, die unter den folgenden Gruppen

2-Pyridyl, 2-(4-Methyl-pyridyl), 2-(6-Methyl-pyridyl), 2-Pyrimidinyl, 5-Methyl-3-isoxazolyl, 2-Thiazolyl, 4-Methyl-2-thiazolyl, 5-Methyl-2-thiazolyl, 4,5-Dimethyl-2-thiazolyl, 2-Benzothiazolyl, 3-Isoxazolyl, 5-Isoxazolyl und 5-Methyl-1,3,4-thiadiazolyl ausgewählt ist.

7. Verfahren nach Anspruch 1, dadurch gekennzeichnet, dass eine Verbindung der allgemeinen Formel (I) hergestellt wird, die unter

5-Äthyl-3-(2-pyridyl)-2H,5H-1,3-oxazino[5,6-c] [1,2]benzothiazin-2,4-(3H)dion-6,6-dioxid,

5-Methyl-3-(2-pyridyl)-2H,5H-1,3-oxazino[5,6-c] [1,2]benzothiazin-2,4-(3H)dion-6,6-dioxid,

5-Methyl-3-(2-pyridyl)-2H,5H-1,3-oxazino[5,6-c] [1,2]benzothiazin-2-(3H)thion-4-on-6,6-dioxid,

5-Methyl-3-[3-(5-methyl-isoxazolyl)]-2H,5H-1,3-oxazino[5,6-c] [1,2]benzothiazin-2,4-(3H)dion-6,6-dioxid,

5-Methyl-3-(2-pyrimidinyl)-2H,5H-1,3-oxazino[5,6-c] [1,2]benzothiazin-2,4-(3H)dion-6,6-dioxid,

5-Methyl-3-[2-(4-methyl-pyridyl)]-2H,5H-1,3-oxazino-[5,6-c] [1,2]benzothiazin-2,4-(3H)dion-6,6-dioxid und

5-Methyl-3-[2-(6-methyl-pyridyl)]-2H,5H-1,3-oxazino[5,6-c] [1,2]benzothiazin-2,4-(3H)dion-6,6-dioxid ausgewählt ist.

**Claims for the Contracting States: BE, CH, DE, FR, GB, IT, LI, LU, NL, SE**

1. New oxazinobenzothiazine 6,6-dioxide derivatives corresponding to the general formula I:

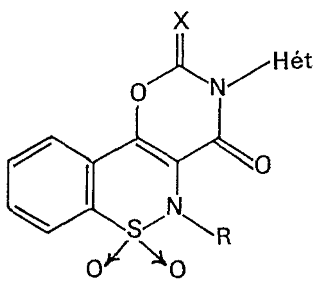

(I)

in which:

R denotes a linear or branched $C_1$ to $C_4$ lower alkyl radical, especially methyl and ethyl radicals,

X denotes an oxygen or sulphur atom,

Het denotes a mono- or bicyclic unsaturated heterocyclic system containing a nitrogen hetero atom, in the ortho-position to which atom the said heterocyclic system is joined to the nitrogen atom of the oxazine ring, the said heterocyclic system optionally containing 1 or 2 other additional hetero atoms chosen from nitrogen, oxygen and sulphur, and being capable, in addition, of being substituted with one or two methyl radicals.

2. New derivatives of general formula I according to Claim 1, characterized in that Het denotes an unsaturated 5- or 6membered monocyclic heterocyclic radical.

3. New derivatives of general formula I according to one of Claims 1 or 2, characterized in that Het denotes a heterocyclic radical chosen from the following radicals:

2-pyridyl; 2-(4-methylpyridyl); 2-(6-methylpyridyl); 2-pyrimidinyl; 5-methyl-3-isoxazolyl; 2-thiazolyl, 4-methyl-2-thiazolyl; 5-methyl-2-thiazolyl; 4,5-dimethyl-2-thiazolyl; 2-benzothiazolyl; 3-isoxazolyl; 5-isoxazolyl; 5-methyl-1,3,4-thiadiazolyl.

4. New derivatives of the general formula I according to one of Claims 1 to 3, characterized in that they are chosen from:

5-ethyl-3-(2-pyridyl)-2H,5H-1,3-oxazino[5,6-c] [1,2]benzothiazine-2,4(3H)-dione 6,6-dioxide;

5-methyl-3-(2-pyridyl)-2H,5H-1,3-oxazino[5,6-c] [1,2]benzothiazine-2,4(3H)-dione 6,6-dioxide;

5-methyl-3-(2-pyridyl)-2H,5H-1,3-oxazino[5,6-c] [1,2]benzothiazine-2(3H)-thione-4-one 6,6-dioxide;

5-methyl-3-[3-(5-methylisoxazolyl)]-2H,5H-1,3-oxazino-[5,6-c] [1,2]benzothiazine-2,4(3H)-dione 6,6-dioxide;

5-methyl-3-(2-pyrimidinyl)-2H,5H-1,3-oxazino[5,6-c] [1,2]benzothiazine-2,4(3H)-dione 6,6-dioxide;

5-methyl-3-[2-(4-methylpyridyl)]-2H,5H-1,3-oxazino[5,6-c] [1,2]benzothiazine-2,4-(3H)-dione 6,6-dioxide and

5-methyl-3-[2-(6-methylpyridyl)]-2H,5H-1,3-oxazino[5,6-c] [1,2]benzothiazine-2,4(3H)-dione 6,6-dioxide.

5. Process for preparing derivatives of general formula I according to one of Claims 1 to 4, characterized in that a compound of general formula II

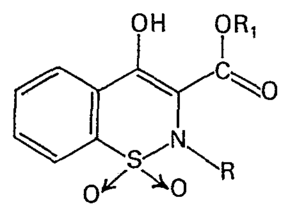

(II)

in which R and $R_1$ denote, independently of each other, a linear or branched $C_1$ to $C_4$ lower alkyl radical, especially methyl and ethyl radicals, is reacted with an intermediate compound, prepared in situ, corresponding to the general formula III

$$[Het - N = C = O] \qquad (III)$$

in which Het has the meaning given in one of Claims 1 to 3 in respect of the general formula I.

6. Process according to Claim 5, characterized in that the intermediate compound of general formula III is prepared in situ by thermal decomposition of a urethane derivative of general formula IV

$$Hét - NH - \overset{\displaystyle O}{\overset{\|}{C}} - O - R_2 \qquad (IV)$$

in which:

Het has the meaning given in one of Claims 1 to 3 in respect of the general formula I, and

$R_2$ denotes a phenyl, ethyl, propyl, isopropyl, n-butyl, isobutyl or tert-butyl radical.

7. Process according to one of Claims 5 and 6, characterized in that, to obtain a derivative of the general formula I in which X denotes a sulphur atom, a compound of general formula I in which X denotes an oxygen atom is reacted with a thiating agent.

8. Process according to Claim 7, characterized in that the thiating agent is chosen from $P_2S_5$ and 2,4-bis(4-methoxyphenyl)-2,4-dithioxo-1,3,2,4-dithiadiphosphetane.

9. By way of new drugs, the derivatives of general formula I according to one of Claims 1 to 4.

10. Pharmaceutical compositions, characterized in that they contain, in addition to a pharmaceutically acceptable carrier, an active principle comprising at least one derivative of the general formula I according to one of Claims 1 to 4.

**Claims for the Contracting State: AT**

1. Process for preparing oxazinobenzothiazine 6,6-dioxide derivatives corresponding to the general formula I:

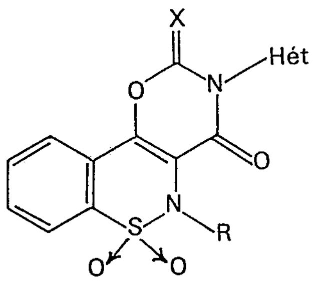

(I)

in which:

R denotes a linear or branched $C_1$ to $C_4$ lower alkyl radical, especially methyl and ethyl radicals,

X denotes an oxygen or sulphur atom,

Het denotes a mono- or bicyclic unsaturated heterocyclic system containing a nitrogen hetero atom, in the ortho-position to which atom the said heterocyclic system is joined to the nitrogen atom of the oxazine ring, the said heterocyclic system optionally containing 1 or 2 other additional hetero atoms chosen from nitrogen, oxygen and sulphur, and being capable, in addition, of being substituted with one or two methyl radicals, characterized in that a compound of general formula II: in which R and $R_1$ denote, indepen-

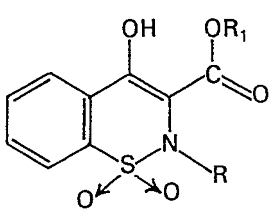

(II)

dently of each other, a linear or branched $C_1$ to $C_4$ lower alkyl radical, especially methyl and ethyl radicals, is reacted with an intermediate compound, prepared in situ, corresponding to the general formula III:

$$[Het - N = C = O] \qquad (III)$$

in which Het has the meaning given above.

2. Process according to Claim 1, characterized in that the intermediate compound of general formula III is prepared in situ by thermal decomposition of a urethane derivative of general formula IV

$$Hét - NH - \overset{\displaystyle O}{\overset{\|}{C}} - O - R_2 \qquad (IV)$$

in which:

Het has the meaning given in Claim 1 in respect of the general formula I, and

$R_2$ denotes a phenyl, ethyl, propyl, isopropyl, n-butyl, isobutyl or tert-butyl radical.

3. Process according to one of Claims 1 and 2, characterized in that, to obtain a derivative of the general formula I in which X denotes a sulphur atom, a compound of general formula I in which X denotes an oxygen atom is reacted with a thiating agent.

4. Process according to Claim 3, characterized in that the thiating agent is chosen from $P_2S_5$ and 2,4-bis(4-methoxyphenyl)-2,4-dithioxo-1,3,2,4-dithiadiphosphetane.

5. Process according to Claim 1, characterized in that a compound of the general formula I is prepared in which Het denotes an unsaturated 5- or 6membered monocyclic heterocyclic radical.

6. Process according to Claim 1, characterized in that a compound of general formula I is prepared in which Het denotes a heterocyclic radical chosen from the following radicals:

2-pyridyl; 2-(4-methylpyridyl); 2-(6-methylpyridyl); 2-pyrimidinyl; 5-methyl-3-isoxazolyl; 2-thiazolyl, 4-methyl-2-thiazolyl; 5-methyl-2-thiazolyl; 4,5-dimethyl-2-thiazolyl; 2-benzothiazolyl; 3-isoxazolyl; 5-isoxazolyl; 5-methyl-1,3,4-thiadiazolyl.

7. Process according to Claim 1, characterized in that there is prepared a compound of general formula I chosen from:

5-ethyl-3-(2-pyridyl)-2H,5H-1,3-oxazino[5,6-c] [1,2]benzothiazine-2,4(3H)-dione 6,6-dioxide;

5-methyl-3-(2-pyridyl)-2H,5H-1,3-oxazino[5,6-c] [1,2]benzothiazine-2,4(3H)-dione 6,6-dioxide;

5-methyl-3-(2-pyridyl)-2H,5H-1,3-oxazino[5,6-c] [1,2]benzothiazine-2(3H)-thione-4-one 6,6-dioxide;

5-methyl-3-[3-(5-methylisoxazolyl)-2H,5H-1,3-oxazino[5,6-c] [1,2]benzothiazine-2,4-(3H)-dione 6,6-dioxide;

5-methyl-3-(2-pyrimidinyl)-2H,5H-1,3-oxazino[5,6-c] [1,2]benzothiazine-2,4(3H)-dione 6,6-dioxide;

5-methyl-3-[2-(4-methylpyridyl)]-2H,5H-1,3-oxazino[5,6-c] [1,2]benzothiazine-2,4(3H)-dione 6,6-dioxide and

5-methyl-3-[2-(6-methylpyridyl)]-2H,5H-1,3-oxazino[5,6-c] [1,2]benzothiazine-2,4(3H)-dione 6,6-dioxide.